# EUROPEAN PATENT APPLICATION

(11) **EP 4 356 816 A1**
(43) Date of publication of application: **24.04.2024**
(21) Application number: 22202620.5
(22) Date of filing: 19.10.2022
(51) Int. Cl.: A61B 3/00

(54) **OPHTHALMOLOGICAL APPARATUS COMPRISING A MAGNETIC CHIN REST**

(71) Applicant: Carl Zeiss Vision International GmbH, 73430 Aalen (DE)
(72) Inventor: Koschorrek, Annika, 89542 Herbrechtingen (DE); Keim, Axel, 89077 Ulm (DE)
(74) Representative: Carl Zeiss AG - Patentabteilung

(57) **Abstract**

The present invention is directed to an ophthalmological apparatus (1) comprising a chin rest (2), characterized in that the chin rest (2) is configured to be releasably mountable on the apparatus (1) via magnetic attachment. Further, the present invention also relates to a chin rest (2) comprising a magnetic element (4).

## Description

### FIELD OF THE INVENTION

The present invention relates to an ophthalmological apparatus and a chin rest thereof using magnetic attachment.

### BACKGROUND

Ophthalmological apparatuses for eye measurement, eye examination or eye treatment have a chin rest to stabilize and fix a subject's head at a proper location during examination, measurement, or treatment of the subject eye. This is to align the subject's eye at a proper position (e.g., centration for eye examination in autorefractor) by adjusting the head position. The chin rest together with a forehead rest determines and adjusts the positioning of the head with respect to the apparatus by movement in vertical direction so that the head is properly placed and fixed between the chin rest and the forehead rest during the operation.

Generally, a chin rest is a unit connected to and motorized by a chin rest support. In this regard, ophthalmological apparatuses already in the market are designed to have a chin rest that is substantially an integral part of the apparatus. This means that the chin rest is an integral part of the apparatus or almost permanently attached to the chin rest support by an interference fit, a screwed connection, etc., requiring considerable amount of force or manipulation for detachment. Most of the chin rest supports are motorized so that they can move vertically.

For instance, Zeiss i. Profiler provides a chin rest as an integral part of the apparatus itself, the chin rest forming a single integral unit with the chin rest support. In Huvitz Auto Ref-Keratometer HRK-9000A, a chin rest is connected to a chin rest support via an interference fit, which is a form of fastening between two tight fitting mating parts that produces a joint held by friction after the parts are pushed together. Therefore, the chin rest cannot be separated from the chin rest support without applying substantial amount of force and dedicating time and effort. Both Topcon KR-800S and Zeiss Visuref 100 use a screwed connection between a chin rest and a chin rest support. The conventional chin rest substantially integrated to the apparatus is difficult to clean and maintain. Due to the recently increased importance of disinfection and sterilization of products or things that frequently go through contact with many different people, a need for a chin rest that is easy to clean and maintain has been increased. CN20763762U takes an approach of using disposable sheath that can be put on the top of the chin rest which is cumbersome and not environment friendly.

In addition, during operation of ophthalmological apparatus, as hand(s) of a subject is likely to be placed near a surface of the apparatus where the chin rest rests on, there is a risk that the subject injures or clamps his/her fingers when the chin rest comes all the way down to the surface of the apparatus. That is, while being attached to the chin rest support, the chin rest will be moved up or down during positioning of a subject's head by adjusting the length of the chin rest support. With a resulting gap between the chin rest and the surface of the ophthalmological apparatus by the height adjustment of the chin rest support, the subject may clamp fingers, for example by placing fingers on the surface of the apparatus where the chin rest will eventually rest on when the chin rest support comes down to the lowest level, the level the same as the surface of the apparatus. In such a case, the gap disappears as the chin rest support comes down near to the same level as the surface of the apparatus and the chin rest meets the surface of the apparatus where the fingers of the subject are frequently placed, thereby causing injury to the subject's hands or fingers. Conventionally, this problem has been solved by design follows function, meaning that the chin rest and the chin rest support are designed to have or maintain a substantial gap from the surface of the ophthalmological apparatus. This kind of injury can be also prevented by using countermeasures such as force detection by the motor in the chinrest, and/or chin rest support or automatic stop at a predetermined distance. However, this technology can be expensive and requires more development and design effort.

### SUMMARY OF THE INVENTION

It is therefore an object of the present invention to provide a chin rest of an ophthalmological apparatus which is easy to clean and maintain, and at the same time can prevent the subject from injury in an economical way.

The purpose of the present invention is achieved by an ophthalmological apparatus and a chin rest thereof, wherein the attachment of the chin rest to the apparatus is realized by magnetic attachment.

### DETAILED DESCRIPTION OF THE INVENTION

The present invention provides an ophthalmological apparatus comprising a chin rest which is releasably mountable on the apparatus via magnetic attachment. The chin rest is configured to be releasably mountable on the chin rest support via magnetic attachment. The chin rest of the present invention can be easily attached to or detached from the apparatus, leading to ease of maintenance and cleaning compared to the conventional devices where chin rest cannot be or at least cannot be easily disassembled from the apparatus.

In the context of the present invention, the term "ophthalmological apparatus" refers to an apparatus for measurement, examination, diagnosis and treatment of diseases and disorders of the eye, for example, biometer, keratometer, topographer, and/or autorefractor.

The term "chin rest" is a part attached to an ophthalmological apparatus to aid in positioning of the subject's chin, ultimately the head, during operation of the apparatus. The chin rest usually has a recess in a shape of a chin (chin cup) so that the subject's head can be firmly and comfortably held by putting the chin on the recess.

The term "chin rest support" is a part of an ophthalmological apparatus configured so that a chin rest can be mounted thereon and/or attached thereto. A chin rest support can be in a form of strut or pillar. A chin rest support can move in a vertical direction (up and down) to adjust the position of the chin rest, leading to a proper positioning of a subject's head. The vertical length of the chin rest support can be adjusted to be shortened and/or extended depending on the proper position of the head of the subject. For example, the chin rest support can be configured to go inside the adjacent part of the ophthalmological apparatus to shorten the length. At its shortest length, the level of the chin rest support becomes the same as the adjacent surface of the ophthalmological apparatus. Generally, the movements of the chin rest support are realized by manual adjustments (with the help of, for example, a joystick or buttons) or using a drive unit, for example, a motor, providing semi- or fully automatic adjustments.

Especially, the magnetic attachment between the chin rest and the chin rest support of the ophthalmological apparatus enables easy and quick detachment of the chin rest from the ophthalmological apparatus. The chin rest releasably mounted on the chin rest support via magnetic attachment is configured to provide a tight attachment to the chin rest support of the apparatus to ensure stable and precise positioning of the subject's head, and further, it also enables the loosening and release of the chin rest from the chin rest support in a case where the subject happens to put the finger between the chin rest and the surface of the ophthalmological apparatus. Thus, in addition to the advantages mentioned above, the chin rest of the present invention therefore can prevent the subject's injury during the operation of the apparatus with a very simple manner.

The goal of the present invention can be achieved by magnetic elements forming magnetic attachment. The present invention provides an ophthalmological apparatus which comprises a chin rest comprising a first magnetic element. In this regard, the present invention also provides a chin rest which comprises a first magnetic element. Further, the ophthalmological apparatus of the present invention comprises a chin support which comprises a second magnetic element. In a further embodiment, the present apparatus can comprise a chin rest comprising a first magnetic element and a chin rest support comprising a second magnetic element.

The term "magnetic element" refers to magnets, which are also referred to as permanent magnet and produce their own persistent magnetic field even in the absence of an applied magnetic field, as well as magnetic materials, which are also referred to as temporary magnet and can produce a magnetic field in response to an applied magnetic field (magnetism), and thus become magnetized in the presence of a magnetic field. Magnetic elements can also be any materials or combinations of materials that are magnetically attracted to one another and generate magnetic field and force. Examples of magnet are neodymium iron boron (NdFeB or NIB), samarium cobalt (SmCo), ferrite (ceramic magnets, e.g., BaFe₂O₃ or SRFe₂O₃) and alnico magnet (Al-Ni-Co). Magnetic materials may be, for example, a ferromagnetic material or ferrimagnetic materials such as irons, iron alloys, or steels.

In one embodiment, the magnetic attachment in the present invention can be realized by the first magnetic element of the chin rest coupled to a second magnetic element of the chin rest support. The coupled magnetic elements generate magnetic field and force, and tract each other, thereby forming the magnetic attachment. The magnetic force refers to magnetic traction between the magnetic elements which provides magnetic attachment.

The magnetic attachment of the present invention is configured to generate a magnetic force that is sufficient to mount and fix the chin rest on the chin rest support so that the subject's head can be stably positioned and fixed on the chin rest, and at the same time allows for easy detachment of the chin rest from the chin rest support. For example, the chin rest is fixed to the chin rest support so that the chin rest is not moved or detached by the force introduced by the subject or subject's chin during the measurement but on the other hand can be released only by applying a force opposite to the direction of the magnetic traction between the chin rest and the chin rest support such as putting a finger between the chin rest and the surface of the ophthalmological apparatus where the chin rest rests on. In other words, the magnetic attachment between the chin rest and the chin rest support can be readily released when a finger is put between the chin rest and the surface of the ophthalmological apparatus. As a result, even before the finger is injured by being clamped between the chin rest and the surface of the ophthalmological apparatus, the chin rest is immediately loosened or released from the chin rest support, and thus the injury of the subject can be easily prevented. This requires neither additional complicated system such as sensor or automated stop nor separate design means for preventing the subject from being injured during the operation of the apparatus such as eye measurement, etc.

In one embodiment, at least one of the first and the second magnetic elements can be a magnet. In another embodiment, the magnet can be selected from the group consisting of neodymium iron boron, samarium cobalt, ferrite and alnico magnet. In a further embodiment, at least one of the first and the second magnetic elements may comprise neodymium iron boron. In another further embodiment, one of the first magnetic element and the second magnetic element comprises neodymium iron boron and the other comprises ferrite. By using neodymium iron boron as a magnetic element, even stronger magnetic attachment which ensures firm and stable holding of the subject's head can be achieved, while still providing detachment of the chin rest by a relatively small force to prevent the subject's injury.

If more stable attachment is required for the operation of the ophthalmological apparatus, the chin rest of the present invention can be configured to comprise additional means for attachment with the ophthalmological apparatus. The term "additional means for attachment" refers to the any attachment between two components except for the magnetic attachment. In a preferred embodiment, the additional means for attachment also provides easy and simple attachment and detachment. For example, the additional means for attachment can be a pair of protrusion and a hole, or a snap joint.

### BRIEF DESCRIPTION OF THE DRAWINGS

Further features, properties and advantages of the present invention will become apparent from the following description of exemplary embodiments of the invention with reference to the drawings.
FIG 1A and 1B are the front view and rear view of a first exemplary embodiment of a chin rest 2 of the present invention.
FIG 2 depicts a first exemplary embodiment of a chin rest support 3 of the present invention where the chin rest 2 illustrated in FIG 1 can be mounted on.
FIG 3A to 3D show a one embodiment of an ophthalmological apparatus 1 for measurement of biometric data of an eye where a chin rest 2 is releasably mounted on the chin rest support 3 of the apparatus 1 via magnetic attachment.
FIG 4 is the rear view of a second exemplary embodiment of a chin rest 20 of the present invention.
FIG 5 depicts a second exemplary embodiment of a chin rest support 30 of the present invention where the chin rest 20 illustrated in FIG 4 can be mounted on.

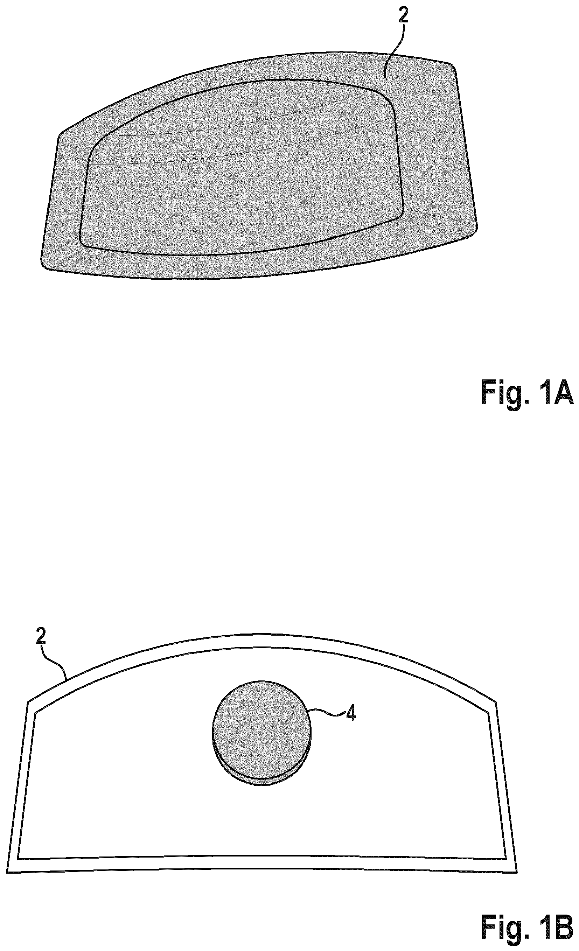

The first exemplary embodiment of the present invention will be described with respect to Figures 1A, 1B, 2 and 3A to 3D. Figure 1A shows a front view and Figure 1B shows a rear view of a first exemplary embodiment of a chin rest 2 according to the present invention. The chin rest 2 is configured to be releasably mounted on a chin rest support 3 of an ophthalmological apparatus 1 via magnetic attachment. The chin rest comprises a first magnetic element 4 for the magnetic attachment.

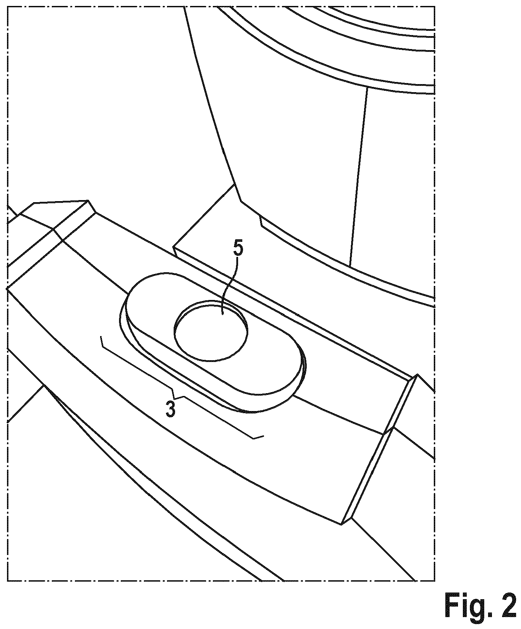

Figure 2 shows a chin rest support 3 on which the chin rest 2 of Figures 1A and 1B is configured to be releasably mounted on. The chin rest support 3 comprises a second magnetic element 5. The first magnetic element 4 is coupled to the second magnetic element 5 and provides magnetic attachment.

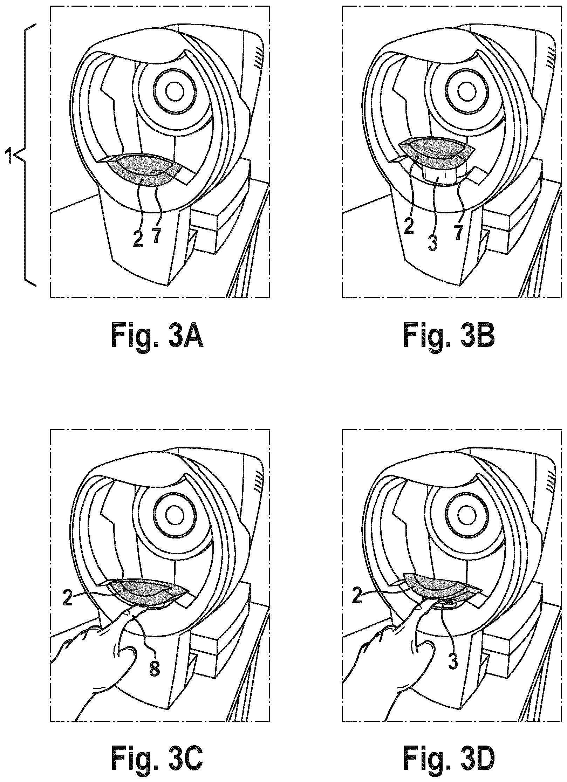

Figure 3 shows an ophthalmological apparatus 1 where the chin rest 2 of Figure 1 is releasably mounted on the chin rest support 3 of Figure 2 via magnetic attachment. The magnetic attachment is generated between the first magnetic element 4 and the second magnetic element 5. The apparatus 1 of Figure 3 is an ophthalmological apparatus for measurement and analysis of biometric data of an eye of a subject. In Figure 3A, the chin rest support 3 is in its shortest length and the upper surface 7 of the ophthalmological apparatus 1 is in a direct contact with the chin rest 2. There is no space or gap between the chin rest 2 and the surface 7 of the ophthalmological apparatus 1. In Figure 3B, the chin rest support 3 is elongated to its maximum height to adjust the positioning of a subject's head, and thus a space is generated between the chin rest support 3 and the surface 7 of the ophthalmological apparatus 1. In Figure 3C, while a finger 8 of the subject is put in the space created between the chin rest 2 and the surface 7 of the ophthalmological apparatus 1, the chin rest support 3 shortens its length, making the chin rest 2 to come down to the lowest level, almost being in a direct contact with the upper surface 7 of the ophthalmological apparatus 1. Here, the finger 8 of the subject is in a risk of being clamped between the chin rest 2 and the surface 7 of the ophthalmological apparatus 1. Figure 3D shows that the chin rest 2 releasably mounted on the chin rest support 3 can be readily detached or loosened from the chin rest support 3 just by the finger lying therebetween, and thus prevents injury of the finger.

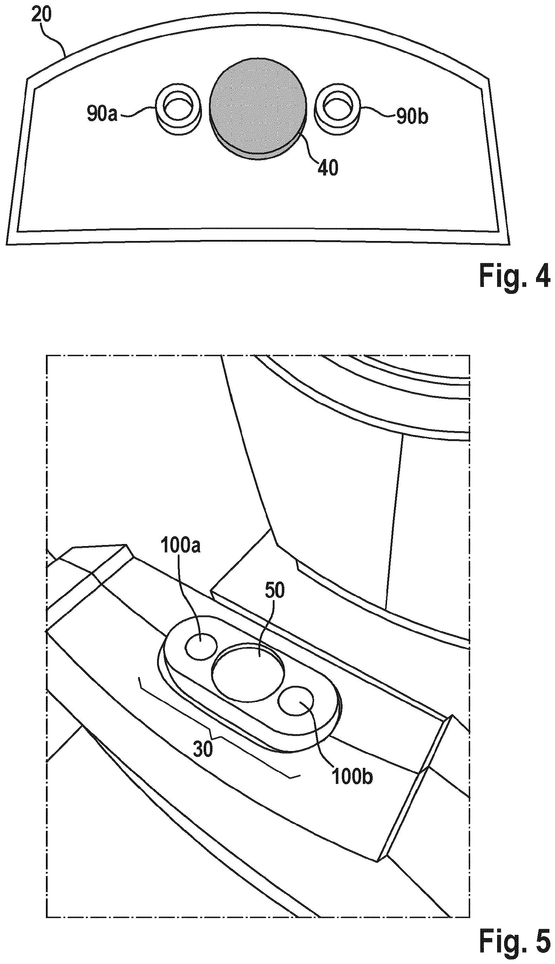

Figures 4 and 5 show a second exemplary embodiment of the present invention where the chin rest 20 and the chin rest support 30 have additional means for attachment 90, 100 with an ophthalmological apparatus 1 other than magnetic attachment provided by the magnetic elements 40, 50. As shown in Figure 4, the chin rest 20 comprises two protruding parts 90a and 90b, and as shown in Figure 5, the chin rest support 30 comprises two holes 100a and 100b matching the protruding parts 90a and 90b. These additional means for attachments can provide more stable fixation of the chin rest 20 to the chin rest support 30 as needed.

## Claims

1. An ophthalmological apparatus (1) comprising a chin rest (2), **characterized in that** the chin rest (2) is configured to be releasably mountable on the apparatus (1) via magnetic attachment.

2. The apparatus (1) according to Claim 1, **characterized in that** the chin rest (2) is configured to be releasably mountable on a chin rest support (3) of the apparatus (1).

3. The apparatus (1) according to Claim 1 or 2, **characterized in that** the chin rest (2) comprises a first magnetic element (4) and/or the chin rest support (3) comprises a second magnetic element (5).

4. The apparatus (1) according to Claim 3, **characterized in that** the first magnetic element (4) of the chin rest (2) is coupled to the second magnetic element (5) of the chin rest support (3).

5. The apparatus (1) according to any one of Claims 3 or 4, **characterized in that** the first magnetic element (4) and/or the second magnetic element (5) comprises a magnet.

6. The apparatus (1) according to Claim 5, **characterized in that** the magnet is selected from the group consisting of neodymium iron boron, samarium cobalt, ferrite and alnico magnet.

7. The apparatus (1) according to any one of Claims 3 to 6, **characterized in that** at least one of the first magnetic element (4) and the second magnetic element (5) is neodymium iron boron.

8. The apparatus (1) according to Claim 3 to 7, **characterized in that** the first magnetic element (4) and the second magnetic element (5) are selected from the group consisting of neodymium iron boron and ferrite.

9. A chin rest (2) for an ophthalmological apparatus (1) **characterized by** comprising a first magnetic element (4).

10. The chin rest (2) according to Claim 9, **characterized in that** the first magnetic element (4) comprises a magnet.

11. The chin rest (2) according to Claim 9 or 10, **characterized in that** the first magnetic element (4) comprises neodymium iron boron.

12. The chin rest (2) according to any one of Claims 9 to 11, **characterized by** further comprising additional means for attachment with an ophthalmological apparatus (1).
